# EUROPEAN PATENT APPLICATION

(11) **EP 4 641 588 A1**
(43) Date of publication of application: **29.10.2025**
(21) Application number: 25172473.8
(22) Date of filing: 25.04.2025
(51) Int. Cl.: G21F 3/02, G21F 3/03, A41D 13/05, A61B 6/10

(54) **RADIATION-PROTECTIVE SHIELD**

(30) Priority: 26.04.2024 EP 24172866
(71) Applicant: Texray AB, 412 92 Göteborg (SE)
(72) Inventor: APELL, Petra, 439 37 ONSALA (SE); GELLERSTEDT, Fredrik, 413 09 GÖTEBORG (SE)
(74) Representative: Brann AB

(57) **Abstract**

1. A wearable radiation-protective shield (10) that has an intended position at the neck of a user is proposed. The radiation-protective shield (10) comprises: a head part (12), wherein the head part (12) is a of sheet-like, pliable, and radiation attenuating first material. The head part (12) has a forward end (18) in the intended position relative to the user, the head part (12) has an outer edge (20) and an inner edge (22), and the radiation-protective shield (10) is arranged to position the inner edge (22) at the neck of the user in the intended position. The head part (12) has a first central line (28) that extends normal to the inner edge (22) and through the corresponding forward end (18) in the planar shape of the head part (12). The head part (12) has a height (h) in a planar shape of the head part (12) between the inner edge (22) and the outer edge (20) and normal to inner edge (22). The head part (12) has a first maximum height (hmax) at a point between the first central line (28) and a first end (34) of the inner edge (22) that is greater than the height at the first central line (28). The head part (12) further has a second maximum height at a point between the first central line (28) and the second end (36) of the inner edge (22), and the second maximum height is greater than the height at the first central line (28).

## Description

### Technical field

The technology proposed herein relates to wearable radiation-protective shields, and particularly to X-ray protective shields protecting the head.

### Background

Ionizing radiation, such as X-ray, is commonly used in diagnostic and surgical procedures, such as in Interventional Radiology (IR). The clinical staff is exposed to the radiation during the procedures. The radiation can be direct or by scattering. Wearable radiation-protective garments, such aprons, thyroid collars are used to reduce the exposure. There is a risk for radiation eye injury due to the radiation exposure during diagnostic and surgical procedures. At the same time, it is advantageous if the vision of the clinical staff is impaired as little as possible by the radiation-protective garments or equipment.

### Object of the proposed technology

The proposed technology aims at reducing radiation exposure towards the eyes with a low to moderate impairment of the effective field vision. It is a further object of the proposed technology to provide a wearable radiation-protective shield that is comfortable to wear.

### Summary

In a first aspect of the proposed technology, a wearable radiation-protective shield, or device, that has an intended position, or intended configuration, on, or at the neck of, a user is proposed. The radiation-protective shield comprises: a head part, or upper part, wherein the head part is a of sheet-like, pliable, and radiation attenuating first material. The head part has a forward end in the intended position relative to the user. The head part has an outer edge, or first edge, and an inner edge, or second edge. The radiation-protective shield is arranged to position the inner edge, or the complete inner edge, at the neck of the user in the intended position. The head part extends upwards from the inner edge to the outer edge in the intended position. The head part has a curved shape, or curved configuration, in the intended position and a planar shape, or planar configuration, if flattened on a planar surface, or at a flattening on a planar surface. The head part has a first head-part section and a second head-part section that are divided by, or along, a first central line that extends normal to the inner edge and through the corresponding forward end in the planar shape of the head part. The outer edge has a first end located on the first head-part section and a second end located on the second head-part section. The inner edge has a first end located on the first head-part section and a second end located on the second head-part section. The head part has a height h in the planar shape of the head part between the inner edge and the outer edge and normal to inner edge. The head part has a first maximum height at a point between the first central line and the first end of the inner edge, and the first maximum height is greater than the height at the first central line.

The head part may have a second maximum height at a point between the first central line and the second end of the inner edge, and the second maximum height is greater than the height at the first central line. The second maximum height may be the same as the first maximum height. Alternatively, the second maximum height may be different from first maximum height. The first maximum height may be greater than the height at the first central line by a factor that is less than 2, less than 1.8, less than 1.6, or less than 1.5. Similarly, the second maximum height may be greater than the height at the first central line by a factor that is less than 2, less than 1.8, less than 1.6, or less than 1.5.

It is understood that the outer edge may be located above, or at a level above, the inner edge in the intended position. It is further understood that the first maximum height is at a point on the first head-part section. Similarly, it is understood that that the second maximum height is at a point on the second head-part section. The proposed geometry of the neck part contributes to reduce radiation exposure to the eyes, for example in IR applications, with little impairment of the effective field of vision of the user. For example, first head-part section may be on the right side of the first central line and the second head-part section may be on the left side of the first central line relative to the user, or vice versa. Worded differently, the first ends of the outer edge and the inner edge may be on the right side of the first central line, and the second ends of the outer edge and the inner edge may be on the left side of the first central line relative to the user, or vice versa.

The radiation-protective shield may be an X-ray protective shield. The radiation attenuating first material may be an X-ray attenuating first material. The X-ray attenuating first material may be arranged to attenuate X-ray radiation with energies at least in the range 50-150 kV. The X-ray attenuating first material may have a 0.25 to 0.50 mm Pb equivalence. The first material may be non-transparent or non-translucent to optical, or visible, light.

It is understood that in the intended position, the user is standing upright with the face facing straight forward. It is further understood that the first maximum height is the greatest height at, or on, the first head-part section. Similarly, it is understood that the second maximum height is the greatest height along the inner edge at, or on, the second head-part section.

It is understood that the forward end of the head part may be defined by, or located at, the outer edge of the head part. The radiation-protective shield may be arranged to position the outer edge at the forward end at the chin of the user in the intended position. The radiation-protective shield may be arranged to position the outer edge at the forward end at the level of the chin of the user in the intended position. The radiation-protective shield may be arranged to position the outer edge at the forward end in front of the chin of the user in the intended position. It is understood that the head part may be arranged to conform to the chin of the user in the intended position. Worded differently, the head part may have a height at the forward end, or along the first central line, adapted to position the outer edge at the forward end at the chin, at the level of the chin, and/or in front of the chin of the user, in the intended position.

The outer edge may be located forward relative to the inner edge at the first central line in the intended position. Worded differently, the head part may, or may be adapted to, extend upwards and outwards relative inner edge, or relative to the neck of the user, at the forward end and in the intended position.

The head part may have a first external angle (α) to a horizontal plane in the range 15° to 45°, 20° to 40°, or 25° to 35° at the forward end or the first central line. It is understood that this is in the intended position, for example with the user standing on the horizontal plane. It is further understood that the radiation-protective shield is between the user and a point within the first external angle. It is further understood that the first external angle may be determined in a plane normal to the head part at the forward end.

The radiation-protective shield may be arranged to position the outer edge on the first head-part section on a first side of the neck of the user in the intended position. Similarly, the radiation-protective shield may be arranged to position the outer edge on the second head-part section on a second side of the neck of the user in the intended position. It is understood that the first side and the second side are opposite relative to the neck of the user. The outer edge may be located above, or directly above, the inner edge at the first side of the neck of the user. The outer edge may be located at neck of the user at the first side of the neck of the user in the intended position. Similarly, the outer edge may be located above, or directly above, the inner edge at the second side of the neck of the user. The outer edge may be located at neck of the user at the second side of the neck of the user in the intended position.

The head part may have a second external angle (β) to a horizontal plane in the range 70° to 100°, or 75 to 95 °, or 80° to 90, at the first side of the neck. Similarly, the head part may have a second external angle (β) to a horizontal plane in the range 70° to 100°, or 75 to 95 °, or 80° to 90, at the second side of the neck. It is understood that this is in the intended position and that the user is standing on the horizontal plane. It is further understood that the radiation-protective shield is between the user and a point within the second external angle. It is further understood that each second external angle may be determined in a plane normal to the head part.

It is understood that the outer edge may outline an outer smooth curve in the planar shape of the head part. It is further understood that the inner edge may outline an inner smooth curve in the planar shape of the head part.

The features described here contributes to the head part extending more outwards at the throat than at the sides of the neck. This contributes to reduce radiation exposure to the eyes, for example in IR applications, with little impairment of the effective field of vision of the user.

The outer edge may have a first outer-edge section that extends from the first central line. It is understood that it extends to a point between the first central line and the first end of the outer edge. The first outer-edge section may be straight or convex in the planar shape of the head part. The outer edge may have a second outer-edge section that extends from the first outer-edge section. It is understood that it extends to a point between the first outer-edge section and the first end of the outer edge. The second outer-edge section may be concave in the planar shape of the head part. The outer edge may have a third outer-edge section that extends from the second outer-edge section. It is understood that it may extend to the first end of the outer edge, or to a point between the second outer-edge section and the first end of the outer edge. The third outer-edge section may be convex in the planar shape of the head part. This contributes to position the outer edge forward below and at the side of the eyes, which improves the radiation protection of the eyes, for example in IR applications, with little impairment of the forward field of vision of the user.

The head part may have a first side edge, or third edge, that interconnects the first end of the outer edge and the first end of the inner edge. Similarly, the head part may have a second side edge, or fourth edge, that interconnects the second end of the outer edge and the second end of the inner edge.

The first side edge and/or the second side edge may be straight. The first side edge and/or the second side edge head part may have a third external angle (γ) to a horizontal plane in the range 60° to 100°, or 70 to 95°, or 80° to 90°. It is understood that this is in the intended position, for example with the user standing on the horizontal plane. It is further understood that a point within each third external angle is located outside the head part. The first side edge and/or the second side edge may be located at the neck of the user in the intended position. Worded differently, the radiation-protective shield may be arranged to position the first side edge and/or the second side edge at the neck of the user in the intended position. The features described here contributes to improve the radiation protection of the eyes, for example in IR applications, with no impairment of the field of vision of the user.

The first maximum height may be greater than the height at the first central line by a factor that is greater than 1.1, greater than 1.2, or greater than 1.3. The inner edge may have a first central-edge section that extends from the first central line to a point between the first central line and the first end of the inner edge. The height across the first central-edge section may be less than 1.1 times the height at the first central line. The inner edge may have a first peripheral-edge section that is located between the first central-edge section and the first end of the inner edge. The height across the first peripheral-edge section may be greater than 1.1, or 1.2, times the height at the first central line. It is understood that the first central-edge section and the first peripheral-edge section may be disjoint. It is further understood that the first maximum height may be along the first peripheral-edge section.

The second maximum height may be greater than the height at the first central line by a factor that is greater than 1.1, greater than 1.2, or greater than 1.3. The inner edge may have a second central-edge section that extends from the first central line to a point between the first central line and the second end of the inner edge. The height across the second central-edge section may be less than 1.1 times the height at the first central line. The inner edge may have a second peripheral-edge section that is located between the second central-edge section and the second end of the inner edge. The height across the second peripheral-edge section may be greater than 1.1, or 1.2, times the height at the first central line. It is understood that the second central-edge section and the second peripheral-edge section may be disjoint. It is further understood that the second maximum height may be along the second peripheral-edge section.

The outer edge on the first head-part section may be between 1.8 and 1.2, 1.7 and 1.3, or 1.6 and 1.4, times longer than the inner edge on the first head-part section.

The first side edge may have a length that is longer than 0.7, 0.8, or 0.9 times the height at the first central line. The first side edge may have a length that is shorter than 1.2, 1.1, or 1.0, times the height at the first central line. Similarly, the second side edge may have a length that is longer than 0.7, 0.8, or 0.9, times the height at the first central line. The second side edge may have a length that is shorter than 1.2, 1.1, or 1.0, times the height at the first central line.

It has been found that the features described here significantly improves the radiation protection of the eyes, for example in IR applications, with little additional impairment of the field of vision of the user.

The first central-edge section may extend to, or at least to, a point having an arc-length separation from the first central line of 3cm, 4 cm, or 5 cm. Similarly, the second central-edge section may extend to, or at least to, a point having an arc-length separation from the first central line of 3cm, 4 cm, or 5 cm.

The first peripheral-edge section may extend from, or at least from, a point having an arc-length separation from the first central line of 4 cm, 5 cm, or 6 cm. Similarly, the second peripheral-edge section may extend from, or at least from, a point having an arc-length separation from the first central line of 4 cm, 5 cm, or 6 cm.

The first peripheral-edge section may extend to, or at least to, a point having an arc-length separation from the first central line of 13 cm, or 15 cm. Similarly, the second peripheral-edge section may extend to, or at least to, a point having an arc-length separation from the first central line of 13 cm, or 15 cm. The first peripheral-edge section may extend to the first end of the inner edge. Similarly, the second peripheral-edge section may extend to the second end of the inner edge.

The inner edge on the first head-part section may be at least 13 cm, or 15 cm, in length. Worded differently, the inner edge on the first head-part section may have an arc length of at least 13 cm, or 15 cm. Similarly, the inner edge on the second head-part section may be at least 13 cm, or 15 cm, in length. Worded differently, the inner edge on the second head-part section may have an arc length of at least 13 cm, or 15 cm.

The first maximum height may be greater than 5 cm, greater than 6 cm, or greater than 7 cm. The first maximum height may be smaller than 9 cm, smaller than 11 cm, or smaller than 13 cm. Similarly, the second maximum height may be greater than 5 cm, greater than 6 cm, or greater than 7 cm. The second maximum height may be smaller than 9 cm, smaller than 11 cm, or smaller than 13 cm.

The first maximum height may be at a point on the inner edge having an arc-length separation from the first central line that is more than 5 cm, more than 6 cm, or more than 7 cm. Similarly, the second maximum height may be at a point on the inner edge having an arc-length separation from the first central line that is more than 5 cm, more than 6 cm, or more than 7 cm.

The first maximum height may be at a point on the inner edge having an arc-length separation from the first central line of that is less than 11 cm, less than 13 cm, or less than 15 cm. Similarly, the second maximum height may be at a point on the inner edge having an arc-length separation from the first central line of that is less than 11 cm, less than 13 cm, or less than 15 cm.

The height at the forward end, or along the first central line, may be greater than 3 cm, greater than 4 cm, or greater than 5 cm. The height at the forward end, or along the first central line, may be smaller than 8 cm, smaller than 9 cm, or smaller than 10 cm.

It has been found that the features described here significantly improves the radiation protection of the eyes, for example in IR applications, with little additional impairment of the field of vision of the user.

The head part may be symmetric relative to the first central line in the planar shape of the head part. More specifically, the first head-part section and a second head-part section may have a reflection symmetry relative to the first central line in the planar shape of the head part. It is specified that the head part has a first head-part section and a second head-part section that are divided by a first central line. Worded differently, the head part may have a bisecting first central line that extends normal to the inner edge and through the corresponding forward end in the planar shape of the head part, wherein the first central line divides the head part in a first head-part section and a second head-part section that are congruent. It is understood that the congruent sections have shapes that are transformable to one another by reflection and/or rotation. The symmetry contributes to an even load on the user, which in extension contributes to a more comfortable radiation-protective shield.

The first material may be, or comprise, a permeable fabric, or fibrous material. The permeable fabric may comprise filaments. The filaments may be arranged in a regular pattern. The filaments may extend in a uniform direction. The uniform direction may be aligned with, or parallel with, the first central line. Worded differently, the uniform direction may be transverse to, or perpendicular to, the outer edge at the forward end. This contributes to a wearable radiation-protection shield that is comfortable and form stable.

Each filament may be of a solid composition that comprises a carrier substance and a radiopaque substance. For example, the solid composition may be a polymeric composite material. The filaments may be arranged in a regular pattern. It is understood that each filament may be a monofilament. It is further understood that the carrier substance and the radiopaque substance may be mixed, and that the radiopaque substance may be evenly distributed within the carrier substance. The carrier substance may comprise at least one polymer and the radiopaque substance may comprise at least one metal. For example, the polymer may be a polyolefin and the metals may be Antimony and Bismuth in the form of powder.

The fabric may be a woven fabric with the filaments forming the weft of the fabric. It is understood that the fabric has a warp, for example of polyester. More specifically, the first material may be any of the radiation-protective material described in WO2014163574A1. The fabric contributes to a more comfortable radiation-protective shield.

Alternatively, the first material may be, or comprise, an impermeable sheet. The impermeable sheet may be of a solid composition that comprises a carrier substance and a radiopaque substance. For example, the solid composition may be a polymeric composite material. It is further understood that the carrier substance and the radiopaque substance may be mixed, and that the radiopaque substance may be evenly distributed within the carrier substance. The carrier substance may comprise at least one polymer and the radiopaque substance may comprise at least one metal.

It is understood that the first material may be composed of several layers, for example two layers of fabric or two impermeable sheets. As specified above, the first material may have a lead equivalence range of 0.25 to 0.50 mm Pb.

The radiation-protective shield may comprise: a head-part lining, or upper part-lining, that covers the head part, or prevent direct access to the head part, wherein the head-part lining is of sheet-like and pliable first lining material. It is understood that the first lining material may be non-attenuating for radiation. For example, the first lining material may be of non-breathable polyurethane or a of fabric of polyester with water-proof breathable membrane.

The head part may have a first attachment part, or upper attachment part, at the inner edge arranged to cooperate with a second attachment part to releasably attach the head part to the second attachment part. For example, the first attachment part and the second attachment part may be a hook-and-loop fastener, or the second attachment part may form a slot into which the first attachment part is inserted. For example, the second attachment part may form part of a radiation-protective neck part or garment.

The head part may be composed of a single piece of the first material. Alternatively, the head part may be composed of a plurality of overlapping pieces of the first material. The overlapping pieces may be joined together. For example, they may be joined together by sewn seams, or construction stiches. Alternatively, the overlapping pieces may be welded or glued together.

The radiation-protective shield may further comprise: a neck part, or middle part, wherein the neck part is a of sheet-like, pliable, and radiation attenuating second material. The neck part is adapted to extend partly, at least partly, or completely around the neck of the user in the intended position. The head part is connected to, or attached to, the neck part at the inner edge of the head part, and the neck part as such has a curved shape, or curved configuration, in the intended position and a planar shape, or planar configuration, if flattened on a planar surface, or at a flattening on a planar surface. It is specified that the neck part is adapted to extend at least partly around the neck of the user in the intended position. Worded differently, the neck part may be adapted to extend from, or at least from, a first side of the neck of the user, across the front of the neck of the user, and to an opposite second side of the neck of the user.

It is understood that it may not be possible to simultaneously flatten the head part and the neck part on a planar surface with the head part connected to the neck part. It is further understood that the head part may extend upwards relative to the neck part in the intended position. It is further understood that the neck part may be arranged to conform to the neck of the user in the intended position. It is further understood that the head part may be fully supported by the neck part in the intended position.

The radiation-protective shield may have a neck-part extension that is connected, or attached, to and extends from the neck part. The neck part and the neck-part extension may be arranged to jointly encircle, or extend completely around, the neck of the user in the intended position. The neck-part extension may be an extension of the neck-part lining described below.

The neck-part extension may have an overlap in the intended position, for example at the back of the neck of the user. The radiation-protective shield, or the neck-part extension, may have a lock at the overlap arranged to releasably lock, or fixate, the neck part around the neck of the user in the intended position. For example, the lock may comprise a hook-and-loop fastener or cooperating magnets.

For example, the head part may be connected to the neck part by a sewn seam, or a construction stich. Alternatively, the head part may be welded or glued to the neck part.

The neck part may be elongated, or tape-like structure. The neck part may have a first upper edge, or fifth edge, and a first lower edge, or sixth edge. The first upper edge may outline a straight line in the planar shape of the neck part. Similarly, the first lower edge may outline a straight line in the planar shape of the neck part. The first upper edge and first lower edge may be aligned, or parallel.

The neck part may be arranged to position the outer edge at the forward end of the head part at the chin at the level of the chin, and/or in front of the chin in the intended position.

The neck part may outline a cylindrical geometry in the intended position. The cylindrical geometry may have a cylinder axis that has an angle (δ) to a horizontal plane at 65°, or in the range 55° to 75°, or 60° to 70°. It is understood that this is in the intended position, for example with the user standing on the horizontal plane.

The inner edge, or the complete inner edge, of the head part may be located at the first upper edge of the neck part. The head part and the neck part may overlap at the inner edge, or along the complete inner edge. The head part and the neck part may overlap by less than 10 mm, or less than 5 mm.

It is specified that the radiation-protective shield may be arranged to position the outer edge on the first head-part section on a first side of the neck of the user. More specifically, the neck part may be arranged to position the outer edge on the first head-part section on a first side of the neck of the user. Similarly, it is specified that the radiation-protective shield may be arranged to position the outer edge on the first head-part section on a second side of the neck of the user. More specifically, the neck part may be arranged to position the outer edge on the second head-part section on a second side of the neck of the user.

The head part may have a fourth external angle (ε) to neck part at the forward end, or the first central line, that is in the range 130° to 160°, 135° to 155°, or 140° to 150°. It is understood that the fourth external angle may be in a plane normal to the head part. It is further understood that the radiation-protective shield is between the user and a point within the fourth external angle.

The shape of the head part may conform to the shape of the neck part on the first side of the neck of the user in the intended position. Similarly, the shape of the head part may conform to the shape of the neck part on the second side of the neck of the user in the intended position. The head part may have a fifth external angle (ζ) to the neck part in the range 160° to 190°, 165° to 185°, or 170° to 180°, at the first side of the neck. Similarly, the head part may have a fifth external angle (ζ) to the neck part in the range 160° to 190°, 165° to 185°, or 170° to 180°, at the second side of the neck. It is understood that each fifth external angle may be in a plane normal head part. It is further understood that the radiation-protective shield is between the user and a point within each fifth external angle.

The neck part may position the first side edge and/or the second side edge at the neck of the user in the intended position. The first side edge and/or the second side edge of the head part may have a sixth external angle (η) to the first upper edge of the neck part that is in the range 35° to 75°, 45° to 70°, or 55° to 65°. It is understood that a point within the sixth external angle is located outside the head part and the neck part. It is further understood that the sixth external angle is relative to the head part at the respective side edge.

It is understood that the upper edge of the neck part may be longer than the inner edge of the head part. It is specified that the neck part may be elongated. The neck part may have a length, or average length, in the planar shape of the neck part in the range 35 cm to 50 cm, or 40 cm to 45 cm. It is understood that the length may be along, or parallel with, the first upper edge and/or the first lower edge. The neck part may have a width, or an average width, in the planar shape of the neck part in the range 3.0 cm to 5.0 cm, or 3.5 to 4.5 cm. It is understood that the width may be transverse to, or perpendicular to, the first upper edge and/or the first lower edge. The combined neck part and neck-part extension may have a length in the range 55 cm to 70 cm, or 60 to 65 cm.

Details of the first material are described above. The second material may have any of the features of the first material described above. For example, the second material may be, or comprise, a permeable fabric, or fibrous material. The permeable fabric may comprise filaments. The filaments may be arranged in a regular pattern. The filaments may extend in a uniform direction. The uniform direction may be transverse to, or perpendicular to, the first upper edge and/or the first lower edge of the neck part. This contributed to a stable yet comfortable wearable radiation-protection shield. The second material may be the same as the first material.

The radiation-protective shield may comprise:
a neck-part lining, or middle part-lining, that covers the neck part, or prevent direct access to the neck part, wherein the neck-part lining is of sheet-like and pliable second lining material. It is understood that the second lining material may be non-attenuating to radiation. For example, the second lining material may be of non-breathable polyurethane or a of fabric of polyester with water-proof breathable membrane. The second lining material may be the same as the first lining material.

The neck part may be composed of a single piece of the second material. Alternatively, the neck part may be composed of a plurality of overlapping pieces of the second material. The overlapping pieces may be joined together. For example, they may be joined together by sewn seams, or construction stiches. Alternatively, the overlapping pieces may be welded or glued together.

It is specified that the head part may be connected to, or attached to, the neck part at the inner edge of the head part. The neck part may have a first attachment part arranged to cooperate with a second attachment part to releasably attach the neck part to the second attachment part. For example, the first attachment part and the second attachment part may form a hook-and-loop fastener, or the second attachment part may form a slot into which the first attachment part is inserted. For example, the second attachment part may form part of a radiation-protective chest part or garment.

The radiation-protective shield may further comprise: a chest part, or lower part, wherein the chest part is a of sheet-like, pliable, and radiation attenuating third material. The chest part is adapted to cover an upper part of the chest at the front of the user in the intended position. The neck part is connected to, or attached to, the chest part at the first lower edge of the neck part, and the chest part as such has a curved shape, or curved configuration, in the intended position and a planar shape, or planar configuration, if flattened on a planar surface, or at a flattening on a planar surface.

The neck part and the chest part may jointly form a thyroid shield, or thyroid collar. It is understood that it may not be possible to simultaneously flatten the head part, the neck part, and the chest part on a planar surface with the parts connected. It is further understood that the chest part may extend downwards relative to the neck part in the intended position. It is further understood that the chest part may be arranged to conform to the chest of the user in the intended position. It is further understood that the chest part may be fully supported by the neck part in the intended position.

The chest part may have a second upper edge, or seventh edge, and a second lower edge, or eighth edge. The second upper edge of the chest part may be concave in the planar shape of the neck part. The second lower edge of the chest part may be convex in the planar shape of the neck part. It is understood that the second upper edge of the chest part may outline an upper smooth curve in the planar shape of the chest part. It is further understood that the second lower edge may outline a lower smooth curve in the planar shape of the chest part.

The second upper edge, or the complete second upper edge, of the chest part may be located at the first lower edge of the neck part. The chest part and the neck part may overlap at the second upper edge, or along the complete second upper edge, of the chest part. The chest part and the neck part may overlap by less than 10 mm, or less than 5 mm.

For example, the chest part may be connected to the neck part by a sewn seam, or a construction stich. Alternatively, the chest part may be welded or glued to the neck part.

The chest part may have a first chest-part section and a second chest-part section that are divided by, or along, a second central line that extends normal to the second upper edge of the chest part, and the chest part is symmetric relative to the second central line in the planar shape of the chest part. More specifically, the first chest-part section and a second chest-part section may have a reflection symmetry relative to the second central line in the planar shape of the chest part. Worded differently, the chest part may have a bisecting second central line that extends normal to the second upper edge of the chest part in the planar shape of the chest part, wherein the second central line divides the chest part in a first chest-part section and a second chest-part section that are congruent. It is understood that the congruent sections have shapes that are that are transformable to one another by reflection and/or rotation.

The chest part has a height in the planar shape of the chest part between the second upper edge and the second lower edge of the chest part and normal to second upper edge of the chest part. The height along the second central line, may be greater than 8 cm, greater than 9 cm, or greater than 10 cm. The height at the second central line, may be smaller than 12 cm, smaller than 14 cm, or smaller than 16 cm.

Details of the first material are described above. The third material may have any of the features of the first material described above. For example, the third material may be, or comprise, a permeable fabric, or fibrous material. The permeable fabric may comprise filaments. The filaments may be arranged in a regular pattern. The filaments may extend in a uniform direction. The uniform direction may be transverse to, or perpendicular to, the second central line. Worded differently, the uniform direction may be aligned with, or parallel with, the second lower edge of the chest part at the centre of the second lower edge and in the planar shape of the chest part.

The third material may be the same as the first material and/or the second material.

The radiation-protective shield may comprise:
a chest-part lining, or lower part-lining, that covers the chest part, or prevent direct access to the chest part, wherein the chest-part lining is of sheet-like and pliable third lining material. It is understood that the third lining material may be non-attenuating to radiation. For example, the third lining material may be of non-breathable polyurethane or a of fabric of polyester with water-proof breathable membrane. The third lining material may be the same as the first lining material and/or the second lining material.

The chest part may be composed of a single piece of the third material. Alternatively, the chest part may be composed of a plurality of overlapping pieces of the third material. The overlapping pieces may be joined together. For example, they may be joined together by sewn seams, or construction stiches. Alternatively, the overlapping pieces may be welded or glued together.

### Brief description of the drawings

A more complete understanding of the abovementioned and other features and advantages of the technology proposed herein will be apparent from the following detailed description of preferred embodiments in conjunction with the appended drawings, wherein:
**Figs. 1a and 1b** show an embodiment of a radiation-protective shield in an intended position on a user.
**Figs. 2a to 2d** show different views of the radiation-protective shield of Figs. 1a and 1b.
**Fig. 3** shows the head part of the radiation-protective shield of Figs. 1a and 1b.
**Fig. 4** shows the neck part of the radiation-protective shield of Figs. 1a and 1b in the same scale as in Fig. 3.
**Fig. 5** shows the chest part of the radiation-protective shield of Figs. 1a and 1b in the same scale as in Fig. 3.
**Fig. 6** shows the head part of an alternative embodiment of a radiation-protective shield in the same scale as in Fig. 3.
**Fig. 7** shows a graph from a comparative study.

### Detailed description

In the figures and the below description, the same reference numerals are used for the same or related features.

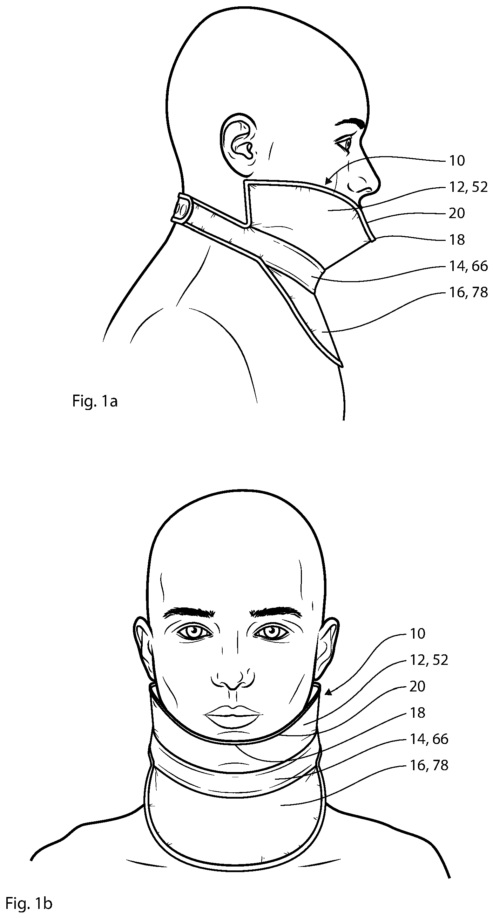

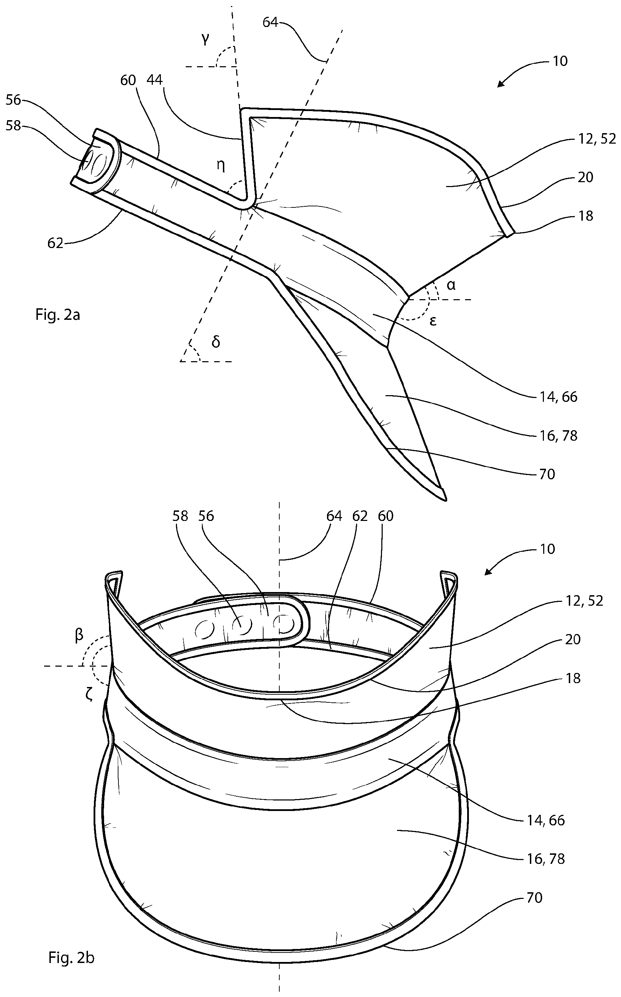

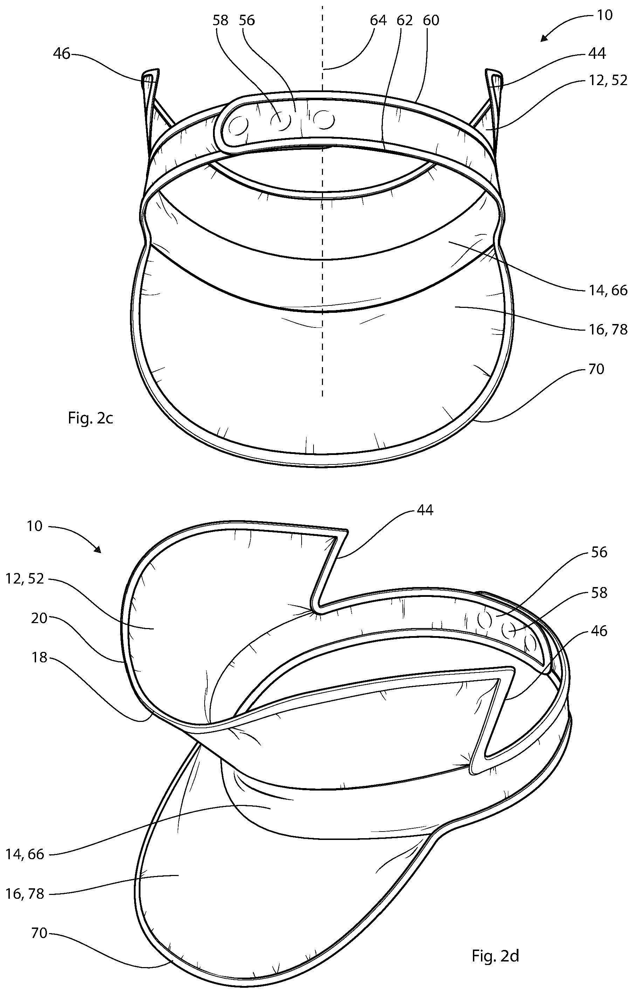

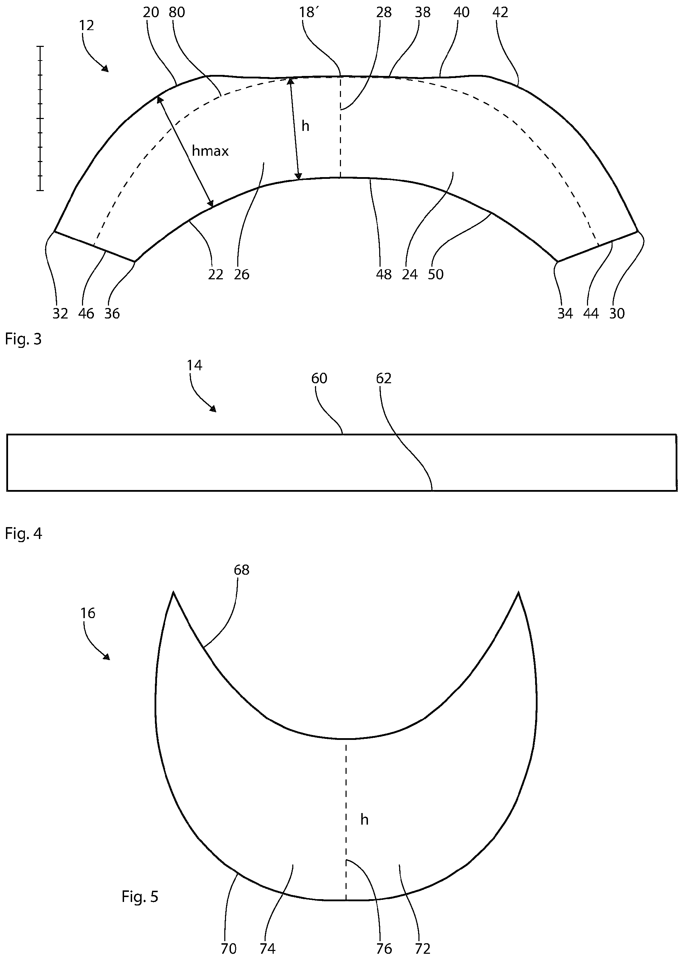

An embodiment of a wearable radiation-protective shield 10 positioned at the neck of a user is shown in **Figs. 1a and 1b****.** The radiation-protective shield 10 is shown in its intended position relative to the user. The user is standing upright on a horizontal surface with the face facing straight forward. Details of the radiation-protective shield 10 are shown in **Figs. 2a to 2d** with the radiation-protective shield 10 in the corresponding intended position during use. The radiation-protective shield has a head part 12, a neck part 14, and a chest part 16 that are connected. Each of the head part 12, neck part 14, and chest part 16 has a curved shape in the intended position, and each of the head part 12, neck part 14, and chest part 16 can be flattened to a planar shape on a planar surface, as shown in **Figs. 3, 4, and 5****.**

The head part 12, neck part 14, and chest part 16 are each a single piece of the same X-ray attenuating material. In alternative embodiments, the different parts 12, 14, and 16 are composite structures of several overlapping pieces of an X-ray attenuating material that are joined together by construction stiches, or that are welded or glued together. In other embodiments, the parts 12, 14, and 16 are of different X-ray attenuating materials.

The X-ray attenuating material can attenuate X-ray radiation with energies at least in the range 50-150 kV. The X-ray attenuating material has a lead equivalent of 0.35 mm Pb equivalence.

The X-ray attenuating material is a permeable fabric that comprises monofilaments arranged in a regular pattern and extending in a uniform direction, for example as described in WO2014163574A1. Each filament is solid composition that comprises a carrier substance of polyolefin and radiopaque substance of Antimony and Bismuth powder that are uniformly mixed. The fabric is a woven fabric with the filaments forming the weft of the cloth and with a warp of polyester. This way, each of the head part 12, neck part 14, and chest part 16, is of a of sheet-like, pliable, and radiation attenuating material, the head part 12 is arranged to conform to the chin of the user, the neck part 12 is arranged to conform to the neck of the user, and the chest part 16 is arranged to conform to the chest of the user, as shown in Figs 1a and 1b.

In alternative embodiments, the first material is an impermeable sheet and/or composed of several layers.

The radiation-protective shield 10 has a head-part lining 52 that covers the head part 12, a neck-part lining 66 that covers the neck part 14, and a chest-part lining 78 that covers the neck part 14. Each lining 52, 66, and 78 prevents direct access to the part 12, 14, and 16 it covers. The linings 52, 66, and 78 are of a fabric of polyester with water-proof breathable membrane.

The head part 12 has an outer edge 20 and an inner edge 22, and the outer edge 20 extends upwards relative to the inner edge 22 in the intended position. The neck part 14 positions the complete inner edge 22 at the neck of the user in the intended position, which is apparent from the combined **Figs. 2a to 2d** **and 3.** The head part 12 extends upwards relative to and is fully supported by the neck part 14 in the intended position. This way, the neck part 14 is arranged to position the outer edge 20 of the head part 12 at the chin of the user in the intended position.

The neck part 14 as such has a curved shape, extends partly around the neck of the user, and conforms to the neck of the user in the intended position, as shown in **Figs. 1a and 1b****.** The neck part 14 has a neck-part extension 56 that is connected to and extends from the neck part 14. The neck-part extension 56 is formed as an extension of the head-part lining 52 and has an overlap in the intended position at the back of the neck of the user, see for example **Figs.** 1a and 1b. The neck-part extension 56 has a lock 58 at the overlap in the form of cooperating magnets that releasably lock the neck part 14 around the neck of the user in the intended position. The combined neck part 14 and neck-part extension 56 has a length of 65 cm.

The head part 12 and the neck part 14 overlap along the complete inner edge 22 by about 5 mm, and the head part 12 is connected to the neck part 14 at the inner edge 22 by a construction stich. In alternative embodiments, the head part 12 is welded or glued to the neck part 14. In its planar shape, the neck part 14 part is elongated and has a straight first upper edge 60 and a straight first lower edge 62 that are parallel, as shown in **Fig. 4****.**

The chest part 16 has a curved shape and covers an upper part of the chest at the front of the user in the intended position, as shown in **Figs. 1a and 1b****.** The chest part 16 extends downwards from and is fully supported by the neck part 14 in the intended position. The neck part 14 is connected to the chest part 16 at the first lower edge 62 of the neck part 14 by a construction stich. The chest part 16 has a concave second upper edge 68 and a convex second lower edge 70 that both outlines a smooth curve in the planar shape, as shown in **Fig. 5****.** The complete second upper edge 68 of the chest part 16 is located at the first lower edge 62 of the neck part 14, which is apparent from combined **Figs 2a to 2d** **and** **5****.** The chest part 16 and the neck part 14 overlap by about 5 mm and are connected by a construction stich along the complete second upper edge 68 of the chest part 16. In alternative embodiments, the chest part 16 is welded or glued to the neck part 14.

The head part 12 has a forward end 18 in the intended position relative to the user, see for example **Figs. 1a and 1b****.** The corresponding forward end 18' in the planar shape is shown in **Fig. 3****.** The head part 12 has a first head-part section 24 and a second head-part section 26 that are divided by a first central line 28 that extends normal to the inner edge 22 and through the corresponding forward end 18' in the planar shape of the head part 12. The outer edge 20 of the head part 12 has a first end 30 located on the first head-part section 24 and a second end 32 located on the second head-part section 26. Similarly, the inner edge 22 of the head part 12 has a first end 34 located on the first head-part section 24 and a second end 36 located on the second head-part section 26. The uniform direction of the filaments of the material of the head part are aligned with the first central line 28 in the planar shape.

The head part 12 is symmetric in the planar shape of the head part 12 with the first head-part section 24 and the second head-part section 26 being congruent and having a reflection symmetry relative to the first central line 28, as shown in **Fig 3****.**

The head part 12 has a straight first side edge 44 that interconnects the first end 30 of the outer edge 20 and the first end 34 of the inner edge 22. Similarly, the head part 12 has a straight second side edge 46 that interconnects the second end 32 of the outer edge 20 and the second end 36 of the inner edge 22. The uniform direction of the filaments in the material of the neck part 14 is transverse to the first upper edge 60 and the first lower edge 62 of the neck part 14.

In the planar form shown in **Fig. 3****,** the outer edge 20 outlines an outer smooth curve and the inner edge 22 outlines an inner smooth curve. The outer edge 20 of the head part 12 has a convex first outer-edge section 38 that extends from the first central line 28, a concave second outer-edge section 40 that extends from the first outer-edge section 38, and a convex third outer-edge section 42 that extends from the second outer-edge section 40 to the first end 34 of the outer edge 20.

The neck part 14 positions the outer edge 20 on the first head-part section 24 on a first side of the neck of the user and the outer edge 20 on the second head-part section 26 on an opposite second side of the neck of the user, as shown in **Fig. 1b****.** The outer edge 20 is located at neck of the user at the first side and at the second side of the neck. The complete inner edge 22 of the head part 12 is located at the neck and first upper edge 60 of the neck part 14.

The chest part 16 has a first chest-part section 72 and a second chest-part section 74 that are divided by a second central line 76 that extends normal to the second upper edge 68 of the chest part 16. The first chest-part section 72 and the second chest-part section 74 are congruent and have a reflection symmetry relative to the second central line 76 in the planar shape of the chest part 16, as shown in **Fig. 5****.**

The head part 12 has a height h in the planar shape of the head part 12 between the inner edge 22 and the outer edge 20 and normal to inner edge 22, see **Fig. 3****.** The height h at the forward end 18 and along the first central line 28 is such that the head part 12 and the neck part 14 jointly positions the outer edge 20 at the forward end 18 at the level of and in front of the chin in the intended position, as shown in **Figs. 1a and 1b****.**

A rule graduated in centimetres is indicated in **Fig. 3****.** The head part 12 has a height along the first central line 28 of about 7 cm. The head part 12 has a first maximum height hmax at a point on the first head-part section 24 between the first central line 28 and the first end 34 of the inner edge 22. The head part 12 further has a second maximum height (not indicated) at a point on the second head-part section 26 between the first central line 28 and the second end 36 of the inner edge 22. The second maximum height is the same as the first maximum height and is located at the corresponding position on the inner edge 22 relative to the first central line 28 due to the symmetry of the head part 12. In an alternative embodiment, the head part 12 is not symmetric relative to the first central line 28, and the second maximum height is different from the first maximum height and is at a point having a different arc length to the first central line 28 along the inner edge 22.

The first maximum height is about 8.5 cm, which means that it is about a factor 1.2 greater than the height at the first central line 28. The first maximum height is at a point that has an arc-length separation from the first central line 28 of about 9 cm.

The inner edge 22 has a first central-edge section 48 that extends from the first central line 28 to a point between the first central line 28 and the first end 34 of the inner edge 22. The first central-edge section 48 extends to about 5 cm along the inner edge 22 from the first central line 28. The height h across the first central-edge section 48 is less than 1.1 times the height at the first central line 28. The inner edge 22 has a first peripheral-edge section 50 that extends from the first central-edge section 48 to about 14 cm along the inner edge 22 from the first central line 28. The height h across the first peripheral-edge section 50 is greater than 1.1 times the height at the first central line 28. The inner edge 22 has a corresponding second central-edge section (not indicated) and second peripheral-edge section (not indicated) between the first central line 28 and the second end 36 of the inner edge 22.

The inner edge 22 on the first head-part section 24 has an arc length of about 17.5 cm and the outer edge 20 on the first head-part section 24 has an arc length of about 25 cm. This means that the outer edge 20 is about 1.4 times longer than the inner edge 22 on the first head-part section 24. The first side edge 44 has a length of about 6 cm, which means that it is shorter than the height at the first central line 28 by a factor of about 0.9.

The elongated neck part 14 has a length along the first upper edge 60 and the first lower edge 62 of about 46 cm. The neck part 14 has a width of about 4 cm transverse to the first upper edge 60 and the first lower edge 62.

The chest part 16 has a height h in the planar shape of the chest part 16 between the second upper edge 68 and the second lower edge 70 of the chest part 16 and normal to second upper edge 68 of the chest part 16, see **Fig. 5****.** The height along the second central line 76 is about 11 cm.

The head part 12 has a first external angle α to a horizontal plane of about 30° in the intended position, as can be seen in **Fig. 2a****.** The head part 12 has a second external angle β to a horizontal plane of about 85° at the first side and the second side of the neck in the intended position, as can be seen in **Fig. 2b****.** The first side edge 44 and the second side edge 46 of the head part 12 has a third external angle γ to a horizontal plane of about 85° in the intended position, as can be seen in **Fig. 2a****.**

The neck part 14 outlines a cylindrical geometry with a cylinder axis 64 that has an angle δ of about 65° to the horizontal plane in the intended position, as shown in **Fig. 2a****.** The head part 12 has a fourth external angle ε to neck part 14 at the forward end 18 that is about 145°. The shape of the head part 12 conforms to the shape of the neck part 14 on the first side and the second side of the neck of the user in the intended position, as shown in **Fig. 1b****.** The head part 12 has a fifth external angle ζ to the neck part 14 of about 175° at the first side and second side of the neck, as shown in **Fig. 2b****.** The first side edge 44 and the second side edge 46 of the head part 12 has a sixth external angle η to the first upper edge 60 of the neck part 14 that is about 60°, as can be seen in **Fig. 2a****.**

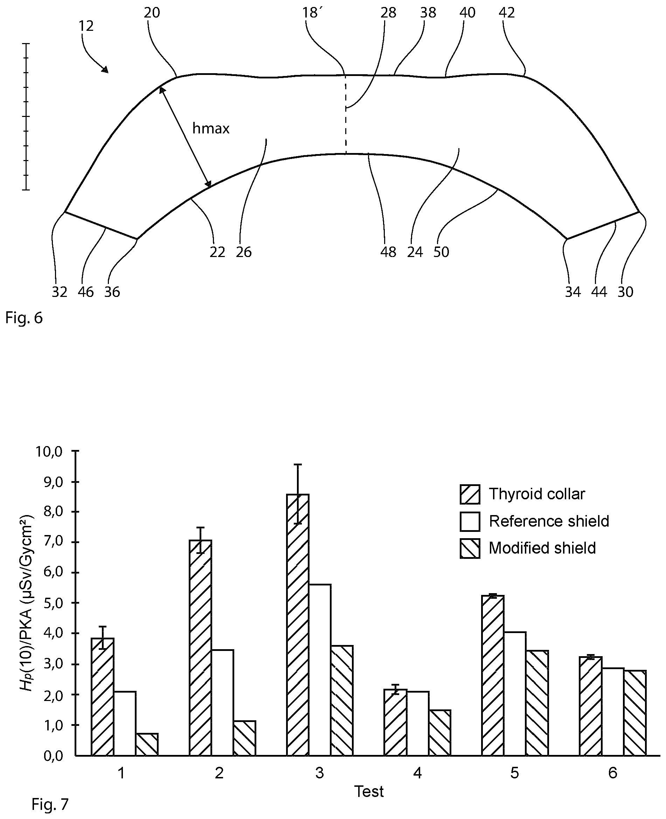

The head part 12 of an alternative embodiment of a radiation-protective shield is shown in **Fig. 6****.** This embodiment shares the features of the radiation-protective shield 10 described in relation Figs. 1 to 5 but differs in the shape and dimensions of the head part 12. The head part 12 has a height along the first central line 28 of about 5.5 cm and the first maximum height hmax is about 7.5 cm, which means that it is about a factor 1.4 greater than the height at the first central line 28. The first maximum height hmax is at a point that has an arc-length separation from the first central line 28 of about 10.5 cm.

The inner edge 22 has a first central-edge section 48 that extends from the first central line 28 to a point between the first central line 28 and the first end 34 of the inner edge 22. The first central-edge section 48 extends to about 5 cm along the inner edge 22 from the first central line 28. The height h across the first central-edge section 48 is less than 1.1 times the height at the first central line 28. The inner edge 22 has a first peripheral-edge section 50 that extends from the first central-edge section 48 to about 15 cm from the first central line 28 along the inner edge 22. The height h across the first peripheral-edge section 50 is greater than 1.1 times the height at the first central line 28. The inner edge 22 has a corresponding second central-edge section (not indicated) and second peripheral-edge section (not indicated) between the first central line 28 and the second end 36 of the inner edge 22.

The inner edge 22 on the first head-part section 24 is the same as in the previous embodiment, which means that it has an arc length of about 17.5 cm. The outer edge 20 on the first head-part section 24 has an arc length of about 24 cm. This means that the outer edge 20 is about 1.4 times longer than the inner edge 22 on the first head-part section 24. The first side edge 44 has a length of about 5 cm, which means that it is shorter than the height at the first central line 28 by a factor of about 0.9.

### Proof-of-concept

A comparative study was performed on a regular thyroid collar, a reference radiation-protective shield (reference shield), and a modified radiation-protective shield (modified shield). The thyroid collar essentially corresponds to the combined neck part 14 and chest part 16 described above in relation to **Figs. 1 to 5****.** The reference shield corresponds to the embodiment described in relation to **Figs. 1 to 5****,** but with head part having the upper edge 80 as outlined by the dashed line in **Fig. 3****.** The height at central line 28 of the reference shield is the maximum height along the inner edge. The modified shield corresponds to the embodiment described in relation to **Figs. 1 to 5****.**

The tests were conducted in a clinical environment on an anthropomorphic phantom 169 cm in height. The phantom was dressed in a radiation protective apron. The thyroid collar, reference shield, and modified shield were positioned on the phantom in the corresponding intended positions on a user. The phantom was arranged in a typical operator position adjacent to an angiography table. The head of the phantom faced the angiography table.

The X-ray imaging equipment (C-arm) was arranged in four different angulations to the vertical (0, 15, 30, 45 °). The focal point of the X-ray beam was on a 26 cm thick PMMA block that had a base area of 25x25 cm2. The purpose of the PMMA block was to simulate a patient. The PMMA block was centered at the C-arm rotation axis (isocenter). The distance from PMMA block to the detector was 100 cm. The phantom was positioned at three different distances from the isocenter (40, 60 and 90 cm). The X-ray tube voltages were 90-94 kV, the currents were 630-800 mA, and the pulse widths were 34-91 ms. A filtration of 0,2-0,5 mm Cu was added to the beams.

The radiation exposure was measured by OSL nanoDots dosimeters at the corresponding left eye position on phantom. The attenuation ratio was estimated as 1-the ratio of the measured µSv/Gycm2 with modified shield and the thyroid collar.

Six different tests were performed on the thyroid collar, reference shield, and modified shield, respectively. The test parameters are presented in the table below.

| Test | Interventionalist position relative to isocenter (cm) | C-arm angle (deg) | kV | mA | Pulse width (ms) | Added filtration (mm Cu) | Field of view (cm) | Focus detector distance (cm) |
|---|---|---|---|---|---|---|---|---|
| 1 | 40 | 15 | 90 | 800 | 34.2 | 0.2 | 40 | 100 |
| 2 | | 45 | 90 | 800 | 34.2 | | | |
| 3 | | 45 | 94 | 630 | 91 | 0.5 | | |
| 4 | 60 | 0 | 90 | 800 | 34.2 | 0.2 | | |
| 5 | | 30 | 90 | 800 | 34.2 | | | |
| 6 | 90 | 0 | 90 | 800 | 34.2 | | | |

The results of the test are presented in the graph of Fig. 7 showing the scatter dose. In a comparison between the modified shield and the reference shield, it can be concluded that the additional features of the head part of the modified shield contribute to an improved the protection of the left eye, particularly in situations corresponding to tests 1 to 4, but also in situations corresponding to tests 5 and 6.

### Item list

### Item list

10 radiation-protective shield
12 head part
14 neck part
16 chest part
18 forward end
20 outer edge of head part
22 inner edge of head part
24 first head-part section
26 second head-part section
28 first central line
30 first end of outer edge
32 second end of outer edge
34 first end of inner edge
36 second end of inner edge
38 first outer-edge section
40 second outer-edge section
42 third outer-edge section
44 first side edge
46 second side edge
48 first central-edge section
50 first peripheral-edge section
52 head-part lining
56 neck-part extension
58 lock
60 first upper edge of neck part
62 first lower edge of neck part
64 cylinder axis
66 neck-part lining
68 second upper edge of chest part
70 second lower edge of chest part
72 first chest-part section
74 second chest-part section
76 second central line
78 chest-part lining
80 outer edge of neck part of reference shield

## Claims

1. A wearable radiation-protective shield (10) that has an intended position at the neck of a user, the radiation-protective shield (10) comprises:
- a head part (12), wherein
the head part (12) is a of sheet-like, pliable, and radiation attenuating first material, the head part (12) has a forward end (18) in the intended position relative to the user, the head part (12) has an outer edge (20) and an inner edge (22), the radiation-protective shield (10) is arranged to position the inner edge (22) at the neck of the user in the intended position, the head part (12) extends upwards from the inner edge (22) to the outer edge (20) in the intended position,
the head part (12) has a curved shape in the intended position and a planar shape if flattened on a planar surface,
the head part (12) has a first head-part section (24) and a second head-part section (26) that are divided by a first central line (28) that extends normal to the inner edge (22) and through the corresponding forward end (18) in the planar shape of the head part (12),
the outer edge (20) has a first end (30) located on the first head-part section (24) and a second end (32) located on the second head-part section (26), the inner edge (22) has a first end (34) located on the first head-part section (24) and a second end (36) located on the second head-part section (26),
the head part (12) has a height (h) in the planar shape of the head part (12) between the inner edge (22) and the outer edge (20) and normal to inner edge (22),
the head part (12) has a first maximum height (hmax) at a point between the first central line (28) and the first end (34) of the inner edge (22), and the first maximum height (hmax) is greater than the height at the first central line (28), and
the head part (12) has a second maximum height at a point between the first central line (28) and the second end (36) of the inner edge (22), and the second maximum height is greater than the height at the first central line (28).

2. The radiation-protective shield (10) according to claim 1, wherein the head part (12) has a first external angle (α) to a horizontal plane in the range 15° to 45°, 20° to 40°, or 25° to 35° at the first central line (28) in the intended position and with the user standing on the horizontal plane.

3. The radiation-protective shield (10) according to claim 1 or 2, wherein the radiation-protective shield (10) is arranged to position the outer edge (20) on the first head-part section (24) on a first side of the neck of the user in the intended position, and the outer edge (20) is located at neck of the user at the first side of the neck in the intended position.

4. The radiation-protective shield (10) according to any of the claims 1 to 3, wherein
the outer edge (20) has a first outer-edge section (38) that extends from the first central line (28), the first outer-edge section (38) is straight or convex in the planar shape of the head part (12),
the outer edge (20) has a second outer-edge section (40) that extends from the first outer-edge section (38), the second outer-edge section (40) is concave in the planar shape of the head part (12),
the outer edge (20) has a third outer-edge section (42) that extends from the second outer-edge section (40) to the first end (30) of the outer edge (20) or to a point between the second outer-edge section (40) and the first end (30) of the outer edge (20), and the third outer-edge section (42) is convex in the planar shape of the head part (12).

5. The radiation-protective shield (10) according to any of the claims 1 to 4, wherein the first maximum height (hmax) is greater than the height at the first central line (28) by a factor that is greater than 1.1, 1.2, or 1.3, and less than 2, 1.8, 1.6, or 1.5.

6. The radiation-protective shield (10) according to any of the claims 1 to 5, wherein the outer edge (20) on the first head-part section (24) is between 1.8 and 1.2, 1.7 and 1.3, or 1.6 and 1.4, times longer than the inner edge (22) on the first head-part section (24).

7. The radiation-protective shield (10) according to any of the claims 1 to 6, wherein the first maximum height (hmax) is greater than 5 cm, 6 cm, or 7 cm, and smaller than 9 cm, 11 cm, or 13 cm.

8. The radiation-protective shield (10) according to any of the claims 1 to 7, wherein the first maximum height (hmax) is at a point having an arc-length separation from the first central line (28) that is more than 5 cm, 6 cm, or 7 cm and less than 11 cm, 13 cm, or 15 cm.

9. The radiation-protective shield (10) according to any of the claims 1 to 8, wherein the height along the first central line (28) is greater than 3 cm, 4 cm, or 5 cm, and smaller than 8 cm, 9 cm, or 10 cm.

10. The radiation-protective shield (10) according to any of the claims 1 to 9, wherein the head part (12) is symmetric relative to the first central line (28) in the planar shape of the head part (12).

11. The radiation-protective shield (10) according to any of the claims 1 to 10, wherein the first material is a permeable fabric that comprises filaments, each filament is of a solid composition that comprises a carrier substance and a radiopaque substance, and the fabric is a woven fabric with the filaments forming the weft of the fabric.

12. The radiation-protective shield (10) according to any of the claims 1 to 11, wherein the radiation-protective shield (10) further comprises:
- a neck part (14), wherein
the neck part (14) is a of sheet-like, pliable, and radiation attenuating second material, the neck part (14) is adapted to extend at least partly around the neck of a user in the intended position, the head part (12) is connected to the neck part (14) at the inner edge (22) of the head part (12), and the neck part (14) as such has a curved shape in the intended position and a planar shape if flattened on a planar surface.

13. The radiation-protective shield (10) according to claim 12, wherein the head part (12) has a fourth external angle (ε) to neck part (14) at the first central line (28) that is in the range 130° to 160°, 135° to 155°, or 140° to 150°.

14. The radiation-protective shield (10) according to claim 12 and 13, wherein the radiation-protective shield (10) is arranged to position the outer edge (20) on the first head-part section (24) on a first side of the neck of the user in the intended position, and the head part (12) has a fifth external angle (ζ) to the neck part (14) in the range 160° to 190°, 165° to 185°, or 170° to 180°, at the first side of the neck.

15. The radiation-protective shield (10) according to any of the claims 12 to 14, wherein the head part (12) has a first side edge (44) that interconnects the first end (30) of the outer edge (20) and the first end (34) of the inner edge (22), the neck part (14) positions the first side edge (44) at the neck of the user in the intended position, and the first side edge (44) of the head part (12) has a sixth external angle (η) to the first upper edge of the neck part (14) that is in the range 35° to 75°, 45° to 70°, or 55° to 65°.
